# EUROPÄISCHE PATENTSCHRIFT

(11) **EP 2 726 845 B1**
(45) Veröffentlichungstag und Bekanntmachung des Hinweises auf die Patenterteilung: **18.11.2015**
(21) Anmeldenummer: 12742895.1
(22) Anmeldetag: 01.08.2012
(51) Int. Cl.: G01N 15/10, G01R 33/12, G01N 15/00

(54) **DYNAMISCHE ZUSTANDSBESTIMMUNG VON ANALYTEN MITTELS MAGNETISCHER DURCHFLUSSMESSUNG**
DETERMINING THE DYNAMIC STATE OF ANALYTES BY MEANS OF MAGNETIC FLOW MEASUREMENT
DÉTERMINATION DYNAMIQUE DE L'ÉTAT D'ANALYTES AU MOYEN D'UNE MESURE MAGNÉTIQUE DE L'ÉCOULEMENT

(30) Priorität: 15.08.2011 DE 102011080945
(43) Veröffentlichungstag der Anmeldung: 07.05.2014
(73) Patentinhaber: Siemens Aktiengesellschaft, 80333 München (DE)
(72) Erfinder: HAYDEN, Oliver, 91074 Herzogenaurach (DE); HELOU, Michael Johannes, 93051 Regensburg (DE); REISBECK, Mathias, 93083 Obertraubling (DE); TEDDE, Sandro, Francesco, 91058 Erlanden (DE)
(86) Internationale Anmeldenummer: PCT/EP2012/064991
(87) Internationale Veröffentlichungsnummer: WO 2013/023910

(56) Entgegenhaltungen:
- WO-A1-2009/068598
- WO-A1-2010/100192
- WO-A2-02/42734
- WO-A2-2008/001261
- US-A1- 2008 003 678
- US-B1- 6 736 978
- JING WU ET AL: "Shear stress mapping in microfluidic devices by optical tweezers", OPTICS EXPRESS, Bd. 18, Nr. 8, 12. April 2010 (2010-04-12) , Seiten 7611-7616, XP55042364, ISSN: 1094-4087, DOI: 10.1364/OE.18.007611

## Beschreibung

Die vorliegende Erfindung betrifft die magnetische Durchflussmessung von magnetisch markierten Zellen.

Im Bereich der Analytmessung und insbesondere der Zellmessung sind zur Bestimmung der Analytgröße und -form bzw. Morphologie Mikroskopie- oder Streulichtverfahren bekannt. Durch Streulichtmessungen, etwa bei der optischen Durchflusszytometrie, wird mittels sogenanntem forward oder side scattering oder der Kombination aus beidem die Zellmorphologie und der Zelldurchmesser detektiert. Streulichtmessungen bedürfen jedoch einer Probenvorbereitung, welche zu Zellstress führen kann, was also die Zelle schon während der Probenvorbereitung verändern bzw. zerstören kann. Somit sind gerade dynamische Veränderungen der zu bestimmenden Größe wie Durchmesser oder Morphologie, die sich innerhalb weniger Minuten abspielen, mit dieser Methode nicht zugänglich.

Mittels mikroskopischen Verfahren ist wiederum eine Zellkonzentrationsbestimmung nicht bzw. nur sehr schwierig zu realisieren. Eine gleichzeitige Erfassung der Zellkonzentration und einer dynamischen Veränderung der Zellform, die auf den Zellzustand hinweist, ist daher mit bisher bekannten Methoden nicht möglich.

Gerade jedoch im Bereich der Analytik in der Diagnostik und in den sogenannten Life Sciences ist es von besonderer Bedeutung aus einer komplexen Suspension, wie etwa einer Blutprobe, Zellen selektiv zu detektieren und deren dynamische Veränderung festzuhalten.

Daher ist es Aufgabe der vorliegenden Erfindung ein geeignetes Verfahren zur dynamischen Erfassung von Zellveränderungen mit der Zeit anzugeben, mit dem auch die Konzentration der Zellen in einer Probe bestimmt werden kann.

Die Aufgabe wird durch ein Verfahren gemäß Patentanspruch 1 gelöst. Eine Vorrichtung zur dynamischen Zustandsbestimmung wird in Patentanspruch 13 angegeben. Vorteilhafte Ausgestaltungen der Erfindung sind Gegenstand der Unteransprüche.

Im Stand der Technik offenbart die Druckschrift US 6,736,978 B1 eine magnetische Durchflussmessung einer Zelle, welche eine Fliessgeschwindigkeitsmessung mithilfe zweier GMR Sensoren beinhaltet. Zellzustände wie Zellmorphologie oder - geometrie werden dabei nicht berücksichtigt.

Bei dem erfindungsgemäßen Verfahren zur magnetischen Durchflussmessung einer Zelle werden folgende Schritte vorgenommen: Zunächst eine magnetische Markierung von Zellen in einer Probe, dann eine Erzeugung eines magnetischen Gradientenfeldes zumindest im Erfassungsbereich einer Sensoranordnung sowie eine Flusserzeugung der Zellen über die Sensoranordnung, wobei der Fluss der Zellen zunächst über ein erstes und anschließend über ein zweites magnetoresistives Bauteil geführt wird. Es erfolgt eine Detektion einzelner markierter Zellen, in der pro Zelle wenigstens drei Messausschläge erfasst werden. Die Messausschläge werden durch das magnetische Streufeld einer jeden markierten Zelle hervorgerufen. Die mindestens drei erfassten Messausschläge bilden ein charakteristisches Messsignal für eine Einzelzelldetektion. Anschließend erfolgt eine Auswertung des Messsignals, bei der anhand der Messausschlagsabfolge das Messsignal als Einzelzelldetektion identifiziert wird und bei der der magnetische Zelldurchmesser anhand des Messausschlagabstandes berechnet wird. Anhand des magnetischen Zelldurchmessers wird dann der Zellzustand bewertet.

Bei dem Verfahren wird also insbesondere ausgenutzt, dass der magnetische Durchmesser, der durch das Streufeldmaximum der markierten Zelle festgelegt wird, kleiner ist als der hydrodynamische oder optische Durchmesser, wie er beispielsweise mit der Coulter-Counter-Methode bestimmt wird. Das bedeutet, dass der Großteil des magnetischen Streufeldes der immunomagnetischen Markierung im Zellinneren verläuft. Die Zellen liegen vorzugsweise in Blutproben vor. Durch die Unterscheidung des optischen zum magnetischen Zelldurchmessers kann der Zellzustand bewertet werden, da bei gleicher Markierung der magnetische Durchmesser mit dem Zustand veränderlich ist.

Insbesondere werden mit dem Verfahren Zellen detektiert, welche wenigstens einen ersten und einen zweiten Zustand annehmen können, wobei sich die beiden Zustände in einer Änderung des magnetischen Zelldurchmessers äußern. Insbesondere bewirkt die Zustandsänderung der Zellen nicht gleichzeitig eine Änderung des hydrodynamischen Zelldurchmessers.

Eine Unterscheidung von abgerundeten und konfluenten Testzellen etwa, war bislang nur mittels aufwendiger Mikroskopie möglich. Mittels der Erfassung des magnetischen Zelldurchmessers kann diese Unterscheidung nun auch in einer Durchflussmessung getroffen werden.

Ein weiteres Beispiel ist die Veränderung eines Thrombozyten durch seine sogenannte Aktivierung. Liegt der Thrombozyt inaktiv als abgeflachtes Ellipsoid vor, meist als Platelet bezeichnet, liegt der magnetische Durchmesser innerhalb der Zelle. Im aktivierten Zustand jedoch, wenn der Thrombozyt eine Vielzahl an Ausstülpungen, den sogenannten Pseudopodien, auf der Oberfläche aufweist, verändert sich dessen magnetischer Durchmesser so, dass dieser sogar außerhalb des hydrodynamischen Durchmessers liegt und diesen bis zu 30 % übersteigt. Das Verfahren hat also den wesentlichen Vorteil zwischen aktivierten und nicht aktivierten Thrombozyten unterscheiden zu können und gleichzeitig deren Konzentration, z.B. in einer stabilisierten Vollblutprobe, bestimmen zu können. Das Verfahren bietet also eine vorteilhafte Möglichkeit zur Thrombozytenfunktionsdiagnostik.

Bei dem Verfahren wird also beispielsweise eine Zustandsänderung bewertet, welche eine Änderung der Zellmorphologie ist und/oder es wird eine Zustandsänderung bewertet, welche auf eine Änderung der Zellgeometrie zurückzuführen ist, wie in den voran genannten Beispielen beschrieben.

In einer vorteilhaften Ausführungsform der Erfindung erfolgen in dem Verfahren mehrere aufeinanderfolgende Messschritte zur Erfassung einzelner Zellen, so dass eine dynamische Veränderung des Zellzustandes anhand seines magnetischen Durchmessers aufgezeichnet wird. Das Verfahren hat also den Vorteil, nicht nur zu einem einzelnen Zeitpunkt einen Zellzustand zu erfassen, sondern diesen über einen Zeitraum hinweg zu verfolgen. Die immunomagnetische Markierung der Zellen wird in dem Verfahren so eingesetzt, dass eine dynamische Veränderung des Zellzustands über einen Zeitbereich von einer Sekunde bis zu einer Stunde beobachtbar ist. Zudem hat das Verfahren den Vorteil, auch die Zellkonzentration in Abhängigkeit von der Zeit zu bestimmen. Beispielsweise sind die mehrere aufeinanderfolgenden Messschritte dadurch realisiert, dass der Fluss der Zellen über mehrere Sensoranordnungen mit je einem ersten und je einen zweiten magnetoresistiven Bauteil geführt wird. Alternativ wird die Probe mehrmals nacheinander über eine oder mehrere Sensoranordnungen geführt.

Das Verfahren birgt also den Vorteil einen Zellzustand nicht nur einmalig erfassen zu können, sondern auch dessen Veränderung mit der Zeit dynamisch zu erfassen und auszuwerten. Gerade dazu trägt auch besonders die magnetische Durchflussmessung an sich bei, da damit kaum eine Probenvorbereitung, außer der Zugabe der magnetischen Marker, vorgenommen werden muss, die die Zellen stressen oder gar zerstören würde. Typischerweise können markierte Zellen mit dem beschriebenen Verfahren im Sekunden- bis Stundenbereich beobachtet werden.

Vorteilhafterweise erfolgt bei dem Verfahren die magnetische Markierung mit magnetischen Nanobeads, insbesondere superparamagnetischen Nanobeads. Diese weisen insbesondere hydrodynamische Durchmesser zwischen 10 nm und 500 nm auf. Von Vorteil sind magnetische Nanobeads, welche als Material Magnetit oder Maghemit aufweisen. Die Markierung erfolgt insbesondere derart, dass die Belegungsdichte der Marker auf der Analytoberfläche, z.B. abhängig von der Zelloberfläche und der Epitopenanzahl auf der Zelloberfläche, zwischen 10% und 90% beträgt. Das Material Magnetit beispielsweise weist eine Sättigungsmagnetisierung von etwa 80 bis 90 (A·m²)/kg auf. Je nach Anteil des Materials oder wenn ein anderes Material in den Nanobeads enthalten ist, weisen die superparamagnetischen Label dann Sättigungsmagnetisierungen zwischen etwa 10 und 60 (A·m²)/kg auf. Eine derartige magnetische Markierung ist von besonderem Vorteil, da dadurch das magnetische Streufeld, welches durch die Markierung im magnetischen Gradientenfeld verursacht wird, überwiegend im Zellinneren verläuft, insbesondere die Maxima des Streufeldes liegen innerhalb des Zelldurchmessers. Dadurch wird von dem Verfahren zusätzlich gewährleistet, dass auch direkt hintereinander folgende Zellen, die sich über die Sensoranordnung bewegen, einzeln ausgelesen werden können. Im Falle eines magnetischen Streufeldes, welches größtenteils außerhalb der Zelle liegt, kommt es zu einer Signalüberlappung des magnetoresistiven Signals, welche keine Eindeutigkeit einer Einzelzelldetektion mehr zulässt.

In einer weiteren vorteilhaften Ausgestaltung der Erfindung wird bei dem Verfahren die Durchflussgeschwindigkeit der Probe so eingestellt, dass die Zellen mit konstanter Geschwindigkeit über die magnetoresistiven Bauteile geführt werden. Insbesondere wird durch die Einstellung der Durchflussgeschwindigkeit beeinflusst, dass die Zellen über den magnetoresistiven Bauteilen abrollen.

In einer weiteren vorteilhaften Ausführungsform der Erfindung erfolgt in dem Verfahren ein Präparationsschritt, bei dem die Kanalinnenwand so modifiziert wird, dass der Zustand der zu detektierenden Zellen bei Kontakt mit der Kanalinnenwand verändert wird. Insbesondere kann es sich bei dieser Zustandsänderung um eine Aktivierung von Zellanalyten handeln. Ein Beispiel dafür sind Thrombozyten. Werden Thrombozyten mit dem beschriebenen Verfahren detektiert, können diese bei Kontakt mit einer geeignet präparierten Kanalinnenwand aktiviert werden, was eine Veränderung der Zellmorphologie zur Folge hat. Wie bereits beschrieben, ändert sich der Thrombozyt dann von einem flachen Ellipsoid zu einem Gebilde mit sehr vielen Ausstülpungen aus der Oberfläche. Eine derartige Zustandsänderung äußert sich eindeutig in dem magnetischen Radius und kann mit dem beschriebenen Verfahren detektiert werden.

Vorzugsweise werden mit dem beschriebenen Verfahren statistische Verteilungen von Durchmessern erfasst. Auch bei nur einer vorliegenden zu detektierenden Zellsorte weisen diese unterschiedliche hydrodynamische Durchmesser sowie unterschiedliche magnetische Durchmesser auf.

Die Vorrichtung zur Durchflussmessung umfasst einen Durchflusskanal, wenigstens eine magnetische Einheit, welche unterhalb des Kanalbodens des Durchflusskanals angeordnet ist und welche ausgestaltet ist ein magnetisches Gradientenfeld zu erzeugen, welches das vom Durchflusskanal umschlossene Volumen durchsetzt, wenigstens eine Zellmesseinrichtung mit wenigstens zwei magnetoresistiven Bauteilen, wobei die magnetische Einheit ausgestaltet ist ein Gradientenfeld mit einer magnetischen Feldstärke zwischen 1 mT und 500 mT hervorzurufen, wobei die magnetoresistiven Bauteile in einem Abstand zwischen 0,2 µm und 40 µm zueinander angeordnet sind und wobei die Kanalinnenwand derart ausgestaltet ist, dass die Adhäsionskraft der Zellen zur Oberfläche der Kanalinnenwand geringer ist als die Scherkraft bei Durchflussgeschwindigkeiten zwischen 0,1 mm/s und 10 mm/s.

In einer vorteilhaften Ausführungsform der Vorrichtung ist der Durchflusskanal hinsichtlich Kanaldurchmesser und Oberflächenbeschaffenheit der Kanalinnenwand so ausgestaltet, dass bei vorgegebenem magnetischen Gradientenfeld und vorgegebener Durchflussgeschwindigkeit auf die Zellen wirkende Scherkräfte geringer sind als 1 g/(cm·s²) und/oder die Reynoldszahl geringer ist als 2000.

Vorteilhafterweise sind die magnetoresistiven Bauteile in einem Maximalabstand zueinander angeordnet, so dass die Zellen die Zellmesseinrichtung im Wesentlichen mit konstanter Geschwindigkeit überstreichen. Der Maximalabstand beträgt insbesondere weniger als 40 µm, beispielsweise weniger als 30 µm. Dadurch ist ein ausreichender Signalhub bei konstantem, externem Magnetfeld gewährleistet.

Bei den magnetoresistiven Bauteilen handelt es sich vorzugsweise um GMR-Sensoren (giant magneto resistance). Die magnetoresistiven Bauteile sind dabei insbesondere in einer Wheatstone'schen Brückenschaltung angeordnet, d.h. die mindestens zwei Magnetowiderstände sind vorzugsweise als Halbbrücke verschaltet. Der vorteilhafte Einsatz einer Wheatstone-Brücke ist beispielsweise aus der Patentanmeldung DE 10 2010 040 391.1 bekannt.

Insbesondere kann die beschriebene Vorrichtung auch eine magnetophoretische Anreicherungsstrecke aufweisen, wie sie beispielsweise aus der Patentanmeldung DE 10 2009 047 801.9 bekannt ist.

Ausführungsformen der vorliegenden Erfindung werden in exemplarischer Weise mit Bezug auf die Figuren 1 bis 8 der angehängten Zeichnung beschrieben. Dabei zeigt:
- Figur 1: eine Seitenansicht der Sensoranordnung,
- Figur 2: ein Diagramm des Messsignals,
- Figur 3: ein Diagramm der Analytverteilung,
- Figur 4: eine schematische Darstellung von inaktiven Thrombozyten,
- Figur 5: eine schematische Darstellung von aktivierten Thrombozyten,
- Figur 6: eine Seitenansicht des Durchflusskanals mit inaktiven Zellen,
- Figur 7: die gleiche Ansicht des Durchflusskanals mit aktivierten Zellen und
- Figur 8: wiederum die gleiche Ansicht des Durchflusskanals, wobei zu den Zellen deren magnetisches Streufeld eingezeichnet ist.

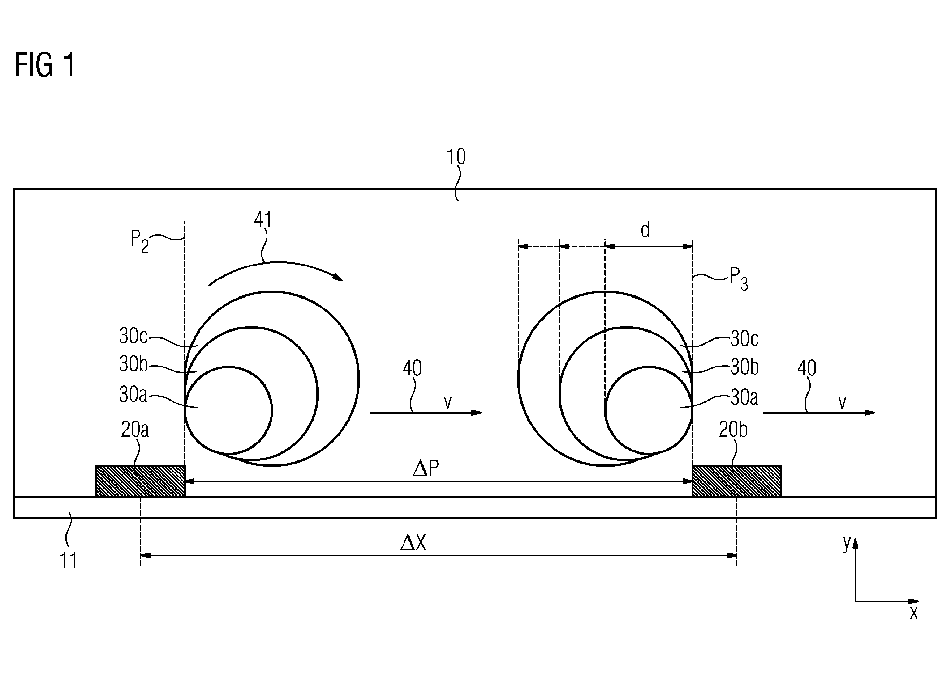

In der Figur 1 ist zunächst ein Ausschnitt eines Durchflusskanals 10 gezeigt, welcher in der Figur von links nach rechts führt. Der Kanalboden 11 des Durchflusskanals 10 trägt zwei stark vereinfacht in der Seitenansicht als Rechtecke dargestellte Magnetsensoren 20a, 20b, die in Durchflussrichtung 40 nacheinander überflossen werden. Die Durchflussrichtung 40 ist durch zwei Geschwindigkeitspfeile v im Durchflusskanal von links nach rechts angezeigt. Die zwei Sensorelemente 20a, 20b befinden sich in einem Mittenabstand Δx voneinander, welcher beispielsweise auf die zu detektierende Analytsorte und insbesondere deren Durchmesser d angepasst sein kann. Im Durchflusskanal 10 sind noch Zellen 30a,b,c als Kreise unterschiedlichen Durchmessers d dargestellt, welche sich in Durchflussrichtung 40 bewegen. Durch einen gekrümmten Pfeil 41 ist angezeigt, dass sich die Zellen 30a,b,c rotierenderweise im Durchfluss 40 fortbewegen. Insbesondere rollen die Zellen 30a,b,c über den Kanalboden 11 ab.

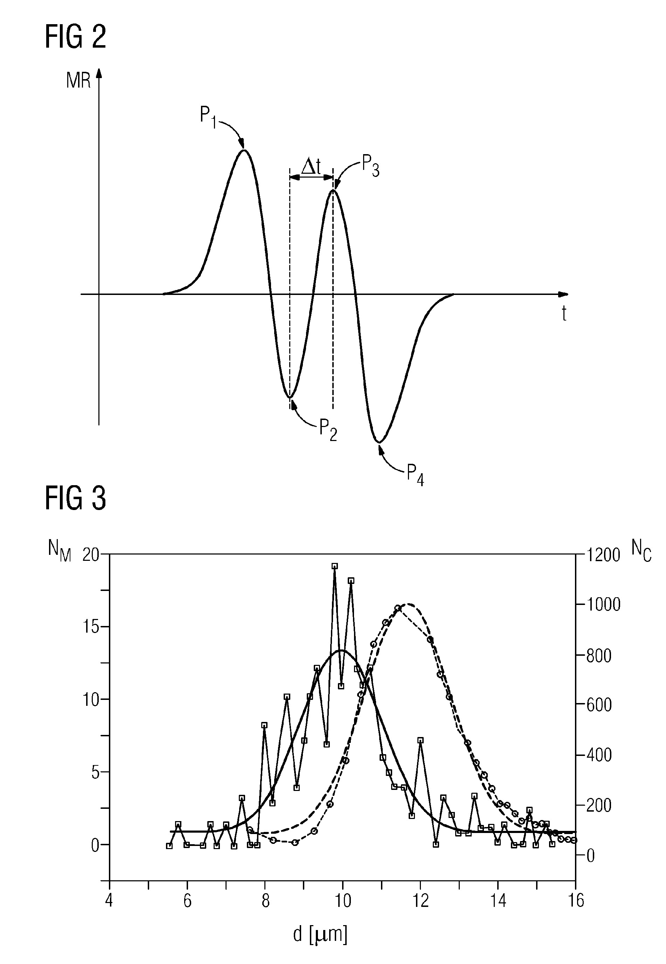

Die Sensorelemente 20a,b,c sind insbesondere magnetoresistive Bauteile, d.h. es handelt sich insbesondere um Magnetowiderstände, welche insbesondere in einer Wheatstone'sche Brückenschaltung miteinander verbunden sind. Bei der Verwendung von zwei Sensoren bilden diese insbesondere eine Halbbrücke. Vorteilhaft an der Verschaltung ist das dadurch erzeugbare magnetoresistive Signal MR, welches in der Figur 2 in einem Signal-Zeit-Diagramm dargestellt ist.

Im zeitlichen Verlauf des Überstreichens einer Zelle 30a,b,c über die beiden Sensorelemente 20a,b wird eine Peak-Abfolge von mindestens drei, beispielsweise aber auch wie in der Figur 2 gezeigt, vier Peaks P1, P2, P3, P4 erzeugt. Dabei entsteht der erste Peak P1, wenn die Zelle 30a,b,c gerade den ersten Sensor 20a erreicht, der zweite Peak P2 entsteht, wenn die Zelle 30a,b,c gerade den ersten Sensor 20a überstrichen hat. Diese Position ist auch in der Figur 1 durch eine senkrecht zum Kanalboden 11 verlaufende gestrichelte Linie P2 angezeigt. Der dritte Peak P3 entsteht dann wenn die Zelle 30a,b,c den zweiten Sensor 20b gerade erreicht, wie ebenfalls wieder durch eine senkrecht zum Kanalboden 11 verlaufende gestrichelte Linie P3 in der Figur 1 gezeigt ist. Der vierte Ausschlag P4 entsteht schließlich wenn die Zelle 30a,b,c auch den zweiten Sensor 20b vollständig überstrichen hat.

D.h., dass der zeitliche Abstand Δt zwischen den zweiten P2 und dritten Peak P3, wie er in der Figur 2 im Diagramm eingezeichnet ist über die Durchflussgeschwindigkeit v mit dem Abstand ΔP der beiden Sensoren 20a,b korreliert. Darüber hinaus ist dieser Peak-Abstand Δt auch davon abhängig, wie groß die Zelle 30a,b,c ist. Je größer der Zelldurchmesser d, desto schneller wird die Zelle 30a,b,c im Durchflusskanal 10 transportiert und desto geringer die Zeit Δt, die zwischen dem zweiten und dritten Peak bzw. den zugehörigen Positionen P2, P3 verstreicht. Mittels des charakteristischen Messsignals MR mit vier Messausschlägen P1 - P4 ist auch eine Einzelanalytdetektion gewährleistet. Die Durchflussgeschwindigkeit v ist beispielsweise aus dem zeitlichen Abstand des ersten P1 und dritten Peaks P3 bestimmbar.

Die Figur 3 schließlich zeigt ein weiteres Diagramm, welches die Verteilung der Zellen um deren gemessenen Durchmesser d zeigt. Der Durchmesser d ist in Mikrometer angegeben. Auf der linken Achse ist die Zellverteilung N_{M} nach dem wie voran beschrieben gemessenen magnetischen Zelldurchmesser d aufgetragen und auf der rechten Achse des Diagramms ist die Zellverteilung N_{c} aufgetragen, welche sich nach dem optischen oder hydrodynamischen Durchmesser des Analyten richtet, welcher insbesondere nach der Coulter-Methode bestimmt wird. Dabei wird sofort ersichtlich, dass die beiden Durchmesser, die durch die magnetische Messung oder durch die Coulter-Messung bestimmt werden, nicht übereinstimmen. Der magnetische Durchmesser liegt in diesem Fall z.B. um etwa 2 µm geringer als der Coulter-Durchmesser. Diese Verschiebung ist darauf zurückzuführen, dass bei Überstreichen der Sensoreinheiten 20a,b nicht der tatsächliche Analyt- bzw. Zelldurchmesser d für die Messausschläge P1 - P4 verantwortlich ist, sondern der Rand 26 des magnetischen Streufeldes 24 bzw. dessen Maximum 26 der x-Komponente des Streufeldes 24, welche von den Sensoren 20a,b erfasst wird.

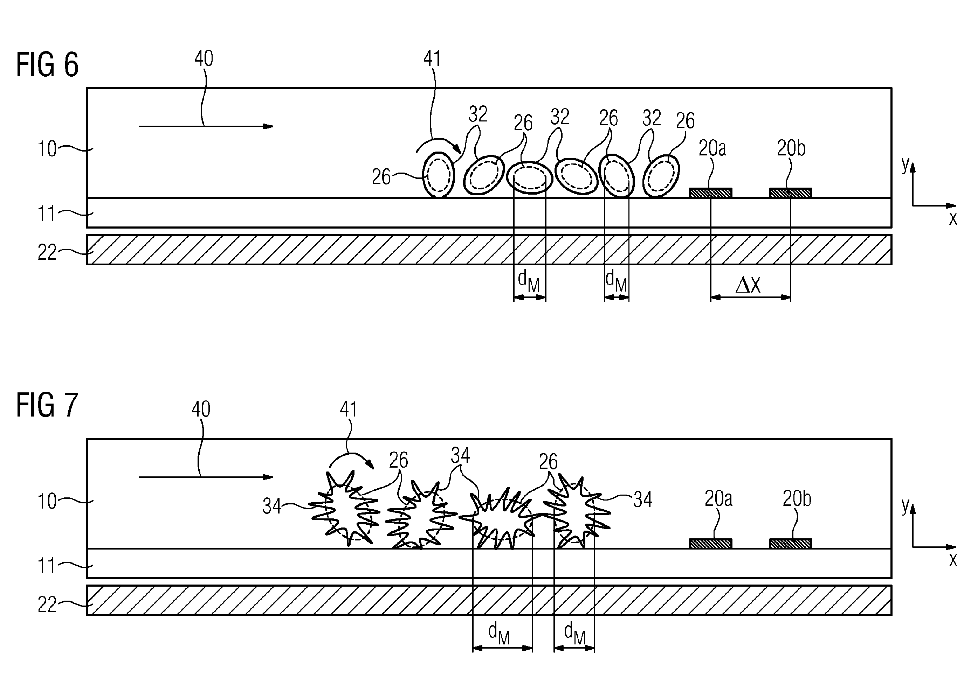

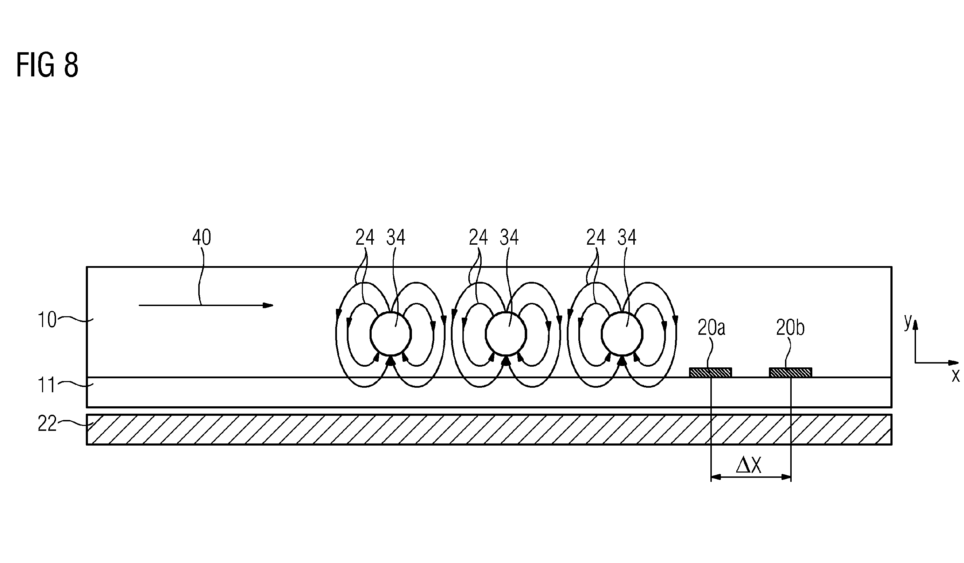

Obwohl die magnetische Markierung außen auf der Zelloberfläche angebracht ist, kann sich das magnetische Streufeld-Maximum auch innerhalb der Zelle 32, 34 befinden. Die Magnetfeldlinie 24 der magnetisch markierten Zellen 32, 34 sind beispielsweise in der Figur 8 gezeigt. Die unterschiedlich liegenden magnetischen Durchmesser d_{M} sind in den Figuren 6 und 7 gezeigt. Dabei spielt nicht nur die magnetische Markierung, sondern besonders auch die Form und Oberfläche des Zellen 32, 34 eine Rolle, ob der magnetische Durchmesser dₘ innerhalb oder außerhalb der zu detektierenden Zelle 32, 34 liegt.

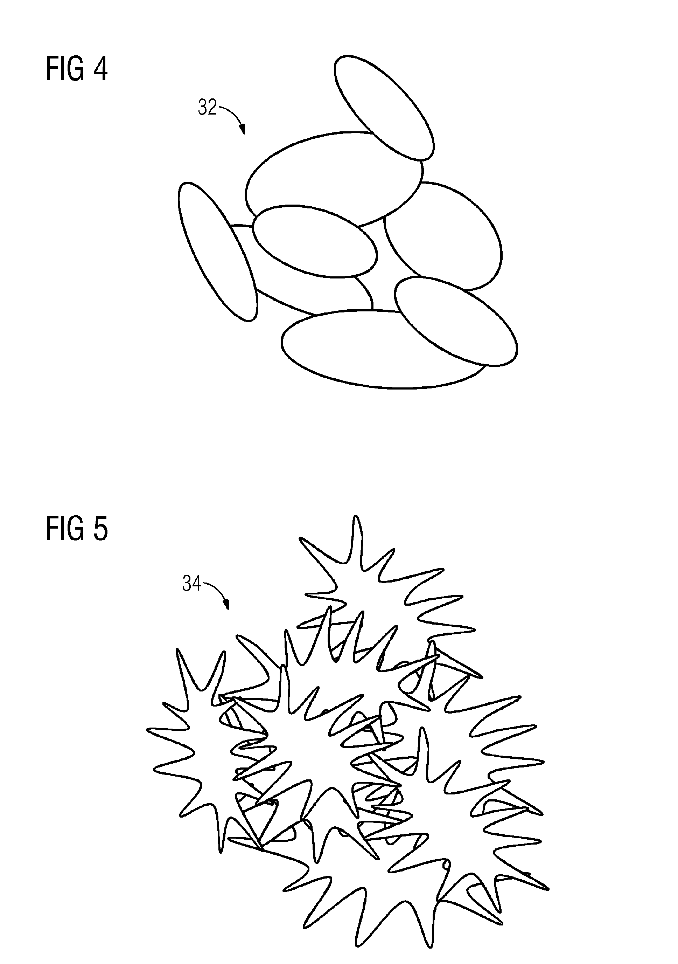

In den Figuren 4 und 5 sind stark vereinfacht schematische Darstellungen von Zellen 32, 34 gezeigt, welche insbesondere Thrombozyten darstellen können. Diese sind in einem ersten Zustand 32 kompakte Ellipsoide wie in Figur 4 dargestellt, in einem zweiten Zustand 34 sind die Thrombozyten sehr unregelmäßig geformt und weisen eine Vielzahl an vorstehenden Ausstülpungen, sogenannten Pseudopodien auf. Diese stellen eine sehr starke Oberflächenvergrößerung der Zelle 34 dar.

Wie in den darauffolgenden Figuren 6 und 7 gezeigt, verändert sich bei gleicher Zelle und gleicher magnetischer Markierung durch die Zustandsänderung von inaktiven Plättchen 32 zu aktivierten Thrombozyten 34 der Durchmesser des magnetischen Streufeldes d_{M}. Bei den kompakten Ellipsoiden 32 liegt dieser innerhalb der Zelle, bei der aktivierten Zelle 34 mit den ausgestülpten Pseudopodien wandert das Maximum 26 des magnetischen Streufeldes 24 nach außen, so dass der magnetische Durchmesser d_{M} größer wird als der tatsächliche Zelldurchmesser d.

In den Figuren 6 bis 8 ist wieder wie in Figur 1 der Durchflusskanal 10 mit dem Kanalboden 11 und der Sensoranordnung 20a,b gezeigt. Unterhalb des Kanalbodens 11 ist die magnetische Einheit 22, welche insbesondere ein Permanentmagnet ist, angeordnet. Dieser ist in seiner flächigen Ausdehnung insbesondere so groß, dass im gesamten Volumen des Durchflusskanals 10 ein homogenes magnetisches Gradientenfeld erzeugt werden kann, welches die magnetisch markierten Zellen 32, 34 am Kanalboden 11 anreichert.

Die Figur 8 schließlich zeigt noch einmal den Querschnitt durch den Durchflusskanal 10 analog zu den Figuren 6 und 7. Eingezeichnet sind magnetisch markierte Zellen 32, 34 und die Magnetfeldlinien 24 des magnetischen Streufeldes, das durch die magnetische Markierung der Zellen 32, 34 erzeugt wird. Von diesem Streufeld 24 detektieren die Sensoren 24a,b, insbesondere die x-Komponente. Die x-Richtung ist durch das Diagramm seitlich des Durchflusskanals 10 angezeigt und weist in Richtung des Durchflusses 40.

## Patentansprüche

1. Verfahren zur magnetischen Durchflussmessung einer Zelle, wobei das Verfahren die folgenden Schritte umfasst:
- magnetische Markierung von Zellen (32, 34) in einer Probe,
- Erzeugen eines magnetischen Gradientenfeldes zumindest im Erfassungsbereich einer Sensoranordnung,
- Flusserzeugung der Zellen (32, 34) über die Sensoranordnung, wobei der Fluss (40) der Zellen (32, 34) zunächst über ein erstes (20a) und anschließend über ein zweites magnetoresistives Bauteil (20b) geführt wird,
**dadurch gekennzeichnet, dass** das Verfahren weiterhin folgende Schritte umfasst:
- Detektion einzelner markierter Zellen (32, 34), indem pro Zelle (32, 34) wenigstens drei Messausschläge der Sensoranordnung (P₁₋₄) erfasst werden, die durch das magnetische Streufeld einer jeden markierten Zelle (32, 34) hervorgerufen werden und welche ein charakteristisches Messsignal für eine Einzelzelldetektion bilden,
- Auswertung des Messsignals, bei der anhand der Messausschlagsabfolge das Messsignal als Einzelzelldetektion identifiziert wird, und bei der der magnetische Zelldurchmesser (26) anhand des Messausschlagabstandes (Δt) berechnet wird , wobei der magnetische Zelldurchmesser (26) durch das Streufeldmaximum der markierten Zelle festgelegt ist, und
- Bewertung des Zellzustandes anhand des magnetischen Zelldurchmessers (26).

2. Verfahren nach Anspruch 1, bei dem eine zu detektierende Zelle (32) wenigstens einen ersten (32) und einen zweiten Zustand (34) annehmen kann, welche sich in einer Änderung des magnetischen Zelldurchmessers (26) äußern.

3. Verfahren nach Anspruch 2, wobei sich die Zustandsänderung nicht gleichzeitig in einer Änderung des hydrodynamischen Zelldurchmessers äußert.

4. Verfahren nach Anspruch 2 oder 3, wobei die Zustandsänderung eine Änderung der Zellmorphologie ist.

5. Verfahren nach Anspruch 2 oder 3, wobei die Zustandsänderung eine Änderung der Zellgeometrie ist.

6. Verfahren nach einem der vorstehenden Ansprüche, bei dem in mehreren aufeinanderfolgenden Messschritten die Erfassung einzelner Zellen (32, 34) erfolgt, so dass eine dynamische Veränderung des Zellzustandes anhand seines magnetischen Durchmessers (26) aufgezeichnet wird.

7. Verfahren nach Anspruch 6, bei dem die mehreren aufeinanderfolgenden Messschritte dadurch realisiert sind, dass der Fluss (40) der Zelle (32, 34) über mehrere Sensoranordnungen mit je einem ersten (20a) und je einem zweiten (20b) magnetoresistiven Bauteil geführt wird.

8. Verfahren nach einem der vorstehenden Ansprüche, bei dem die magnetische Markierung mit magnetischen Nanobeads, insbesondere superparamagnetischen Nanobeads, vorgenommen wird.

9. Verfahren nach einem der vorstehenden Ansprüche, bei dem die Durchflussgeschwindigkeit der Probe so eingestellt wird, dass die Zellen (32, 34) über die magnetoresistiven Bauteile (20a, b) abrollen.

10. Verfahren nach einem der vorstehenden Ansprüche mit einem Präparationsschritt, bei dem die Kanalinnenwand so modifiziert wird, dass der Zustand der zu detektierenden Zellen bei Kontakt mit der Kanalinnenwand verändert wird.

11. Verfahren nach Anspruch 10, wobei es sich bei der Zustandsänderung um eine Aktivierung der Zellen handelt.

12. Verfahren nach Anspruch 10 oder 11, wobei es sich bei den zu detektierenden Zellen (32, 34) um Thrombozyten und bei der Zustandsänderung um eine Aktivierung der Thrombozyten handelt.

## Claims

1. Method for magnetic flow measurement of a cell, the method comprising the following steps:
- magnetic marking of cells (32, 34) in a sample,
- generation of a gradient magnetic field at least in the acquisition region of a sensor arrangement,
- flow generation of the cells (32, 34) over the sensor arrangement, the flow (40) of the cells (32, 34) being guided initially over a first magnetoresistive component (20a) and subsequently over a second magnetoresistive component (20b),
**characterized in that** the method furthermore comprises the following steps:
- detection of individual marked cells (32, 34) by recording, per cell (32, 34), at least three measurement excursions of the sensor arrangement (P₁₋₄) which are induced by the stray magnetic field of each marked cell (32, 34) and which form a characteristic measurement signal for single-cell detection,
- evaluation of the measurement signal, in which the measurement signal is identified as single-cell detection with the aid of the measurement excursion sequence, and in which the cell magnetic diameter (26) is calculated with the aid of the measurement excursion separation **(Δt),** wherein the cell magnetic diameter (26) is established by the stray field maximum of the marked cell, and
- evaluation of the cell state with the aid of the cell magnetic diameter (26).

2. Method according to Claim 1, wherein a cell (32) to be detected can assume at least a first state (32) and a second state (34), which are manifested by a change in the cell magnetic diameter (26).

3. Method according to Claim 2, wherein the state change is not simultaneously manifested by a change in the cell hydrodynamic diameter.

4. Method according to Claim 2 or 3, wherein the state change is a change in the cell morphology.

5. Method according to Claim 2 or 3, wherein the state change is a change in the cell geometry.

6. Method according to one of the preceding claims, wherein the detection of individual cells (32, 34) is carried out in a plurality of successive measurement steps, so that a dynamic change in the cell state is recorded with the aid of its magnetic diameter (26).

7. Method according to Claim 6, wherein the plurality of successive measurement steps are carried out by guiding the flow (40) of the cell (32, 34) over a plurality of sensor arrangements, each having a first magnetoresistive component (20a) and a second magnetoresistive component (20b).

8. Method according to one of the preceding claims, wherein the magnetic marking is carried out with magnetic nanobeads, in particular superparamagnetic nanobeads.

9. Method according to one of the preceding claims, wherein the flow speed of the sample is adjusted in such a way that the cells (32, 34) roll over the magnetoresistive components (20a,b).

10. Method according to one of the preceding claims, having a preparation step in which the channel inner wall is modified in such a way that the state of the cells to be detected is altered upon contact with the channel inner wall.

11. Method according to Claim 10, wherein the state change is activation of the cells.

12. Method according to Claim 10 or 11, wherein the cells (32, 34) to be detected are thrombocytes and the state change is activation of the thrombocytes.

## Revendications

1. Procédé de mesure magnétique de l'écoulement d'une cellule, le procédé comprenant les étapes suivantes :
- marquage magnétique de cellules (32, 34) dans un échantillon,
- production d'un champ à gradient magnétique au moins dans la zone de détection d'un dispositif détecteur,
- génération d'un flux des cellules (32, 34) sur le dispositif détecteur, le flux (40) des cellules (32, 34) étant conduit d'abord sur un premier (20a) et ensuite sur un second composant magnétorésistif (20b),
**caractérisé en ce que** le procédé comprend en outre les étapes suivantes :
- détection de cellules marquées (32, 34) individuelles, en détectant par cellule (32, 34) au moins trois déviations de mesure du dispositif détecteur (P₁₋₄), qui sont provoquées par le champ de dispersion magnétique de chaque cellule marquée (32, 34) et qui forment un signal de mesure caractéristique d'une détection de cellules individuelles,
- exploitation du signal de mesure au cours de laquelle, à l'aide de la séquence des déviations de mesure, le signal de mesure est identifié comme détection de cellules individuelles, et au cours de laquelle le diamètre magnétique de cellule (26) est calculé à l'aide de la distance de déviation de mesure (Δt), le diamètre magnétique de cellule (26) étant défini par le maximum de champ de dispersion de la cellule marquée, et
- évaluation de l'état de cellule à l'aide du diamètre magnétique de cellule (26).

2. Procédé selon la revendication 1, dans lequel une cellule à détecter (32) peut prendre au moins un premier (32) et un second état (34), lesquels se traduisent par un changement du diamètre magnétique de cellule (26).

3. Procédé selon la revendication 2, le changement d'état ne se traduisant pas simultanément par un changement du diamètre hydrodynamique de cellule.

4. Procédé selon la revendication 2 ou 3, le changement d'état étant un changement de la morphologie de cellule.

5. Procédé selon la revendication 2 ou 3, le changement d'état étant un changement de la géométrie de cellule.

6. Procédé selon l'une des revendications précédentes, dans lequel la détection de cellules individuelles (32, 34) est réalisée en plusieurs étapes successives, de sorte à enregistrer une modification dynamique de l'état de cellule à l'aide de son diamètre magnétique (26).

7. Procédé selon la revendication 6, dans lequel lesdites plusieurs étapes successives sont réalisées par conduction du flux (40) des cellules (32, 34) sur plusieurs dispositifs détecteurs ayant chacun un premier (20a) et un second composant magnétorésistif (20b).

8. Procédé selon l'une des revendications précédentes, dans lequel on effectue le marquage magnétique avec des nanobilles magnétiques, en particulier des nanobilles superparamagnétiques.

9. Procédé selon l'une des revendications précédentes, dans lequel on ajuste la vitesse d'écoulement de l'échantillon de façon à faire rouler les cellules (32, 34) sur les composants magnétorésistifs (20a, b).

10. Procédé selon l'une des revendications précédentes comprenant une étape de préparation dans laquelle on modifie la paroi intérieure du canal de façon à changer l'état des cellules à détecter en cas de contact avec ladite paroi.

11. Procédé selon la revendication 10, le changement d'état étant une activation des cellules.

12. Procédé selon la revendication 10 ou 11, les cellules à détecter (32, 34) étant des thrombocytes et le changement d'état une activation des thrombocytes.
